# EUROPÄISCHE PATENTSCHRIFT

(11) **EP 1 418 951 B1**
(45) Veröffentlichungstag und Bekanntmachung des Hinweises auf die Patenterteilung: **20.01.2010**
(21) Anmeldenummer: 02754860.1
(22) Anmeldetag: 10.07.2002
(51) Int. Cl.: A61K 9/70

(54) **TRANSDERMALES THERAPEUTISCHES SYSTEM (TTS) MIT DEM WIRKSTOFF FENTANYL**
TRANSDERMAL THERAPEUTIC SYSTEM (TTS) WITH FENTANYL AS ACTIVE INGREDIENT
SYSTEME THERAPEUTIQUE TRANSDERMIQUE (TTS) CONTENANT DU FENTANYLE COMME PRINCIPE ACTIF

(30) Priorität: 24.08.2001 DE 10141651
(43) Veröffentlichungstag der Anmeldung: 19.05.2004
(73) Patentinhaber: LTS LOHMANN Therapie-Systeme AG, 56626 Andernach (DE)
(72) Erfinder: MÜLLER, Walter, 56626 Andernach (DE)
(74) Vertreter: Schmidt, Werner
(86) Internationale Anmeldenummer: PCT/EP2002/007663
(87) Internationale Veröffentlichungsnummer: WO 2003/018071

(56) Entgegenhaltungen:
- WO-A-01/01967
- WO-A-89/10108
- WO-A-95/18603
- US-B1- 6 221 383

## Beschreibung

Der Gegenstand der vorliegende Erfindung ist ein Transdermales Therapeutisches System (TTS) mit dem Wirkstoff Fentanyl.

Fentanyl und fentanylanaloge Derivate wie Sufentanil, Carfentanyl, Lofentanyl und Alfentanil sind außerordentlich wirksame Analgetika. Ihre geringe Dosierung und ihre physiko-chemischen Eigenschaften wie z. B. der n-Octanol-Wasser-Verteilungskoeffizient, der Schmelzpunkt und das Molekulargewicht machen die transdermale Zufuhr dieser Substanzen in wirksamer Menge möglich, und ihre pharmakokinetischen Eigenschaften wie die schnelle Metabolisierung und der relativ enge therapeutische Index die transdermale Zufuhr wünschenswert.

In der Tat ist seit einigen Jahren ein Transdermales Therapeutisches System (TTS) mit Fentanyl als Wirkstoff auf dem Markt. Dieses System ist ein sogenanntes Reservoirsystem. Unter einem Reservoirsystem wird ein Transdermales Therapeutisches System verstanden, das den Wirkstoff in einer flüssigen oder gelförmigen Zubereitung in einem aus einer undurchlässigen Folie und einer wirkstoffdurchlässigen Membran geformten Beutel enthält. Die undurchlässige Folie dient als Rückschicht, um ein Auslaufen der flüssigen bzw. gelförmigen Wirkstoffzubereitung zur hautabgewandten Seite des Beutels zu verhindern. Die Membran dient zur Kontrolle der Geschwindigkeit der Freisetzung des Wirkstoffs aus der hautzugewandten Seite des Beutels. Auf dieser Seite besitzt die Membran zusätzlich eine Kleberschicht zur Befestigung des gesamten Transdermalen Therapeutischen Systems auf der Haut.

In diesem speziellen Fall (Durogesic^{®}-TTS) ist Fentanyl in einem Gemisch aus Ethanol und Wasser gelöst. Weitere Einzelheiten dieses Systems können der US-Patentschrift 4,588,580 bzw. der DE 35 26 339 entnommen werden, die beide eine detaillierte Beschreibung enthalten.

Reservoirsysteme haben aber einen großen Nachteil, daß nämlich im Falle einer Leckage (z. B. einer einfachen mechanisch bedingten Beschädigung, ein Schnitt oder Riß, ein Aufplatzen der Schweißnaht, etc.) des die wirkstoffhaltige Zubereitung enthaltenden Beutels der Wirkstoff großflächig mit der Haut in Kontakt kommen kann und als Folge davon in zu hohen Dosen resorbiert wird. Dies ist speziell bei Fentanyl und den fentanylanalogen Derivaten lebensgefährlich, da eine Überdosierung sehr schnell zu Atemdepression und damit tödlichen Zwischenfällen führt. Mehrere solcher tödlichen bzw. beinah tödlichen Zwischenfälle sind beschrieben in Clinical Phannacokinet. 2000, 38 (1), 59-89.

WO 01/01967 A offenbart ein transdermales therapeutisches System mit einer für Wirkstoffe undurchlässigen Rückschicht, mindestens einer Polymerschicht mit darin enthaltenen wirkstoffhaltigen Mikroreservoiren und einer vor Gebrauch zu entfernenden Schutzschicht. Dabei besteht der Polymeranteil der Polymerschicht zu mindestens 70 Gew.-% aus Polysiloxanen. Die Mikroreservoire enthalten den Wirkstoff in gelöster Form und bestehen zu mindestens 50 Gew.-% aus einen ambiphilen, dipolaren organischen Lösemittel. Das ambiphile Lösemittel ist zu nicht mehr als etwa 20 Gew.-% in Polysiloxan löslich und vorzugsweise mit Wasser in einem Gewichtsverhältnis von mindestens 1 Teil Lösemittel zu 3 Teilen wasser mischbar. Aufgrund dieser Zusammensetzung ist es möglich, die Beladung von Silikonklebem mit gelösten Wirkstoffen mittlerer Polarität zu verbessern und damit den Einsatzbereich von Silikonklebern zu erweitern.

Aufgabe der vorliegenden Erfindung ist es, ein Transdermales Therapeutisches System mit dem Wirkstoff Fentanyl und / oder fentanylanalogen Derivaten bereitzustellen, das dem Benutzer eine erhöhte Sicherheit bezüglich der versehentlichen Aufnahme von Überdosen bietet.

Die Aufgabe wird gelöst durch ein Transdermales Therapeutisches System (TTS) mit erhöhter Sicherheit hinsichtlich einer versehentlichen Verabreichung einer Überdosis des Wirkstoffes umfassend eine wirkstoffundurchlässige Rückschicht, eine wirkstoffhaltige Schicht, wobei der Wirkstoff Fentanyl, Sufentanil Carfentanyl, Lofentanyl und/oder Alfentanil und/oder ein Salz von Fentanyl, Sufentanil, Carfentanyl, Lofentanyl und/oder Alfentanil ist und wobei die wirkstoffhaltige Schicht ein Polymer oder eine Polymermischung mit darin dispergierten Mikroreservoiren umfasst, eine sich hautseitig anschließende Membranschicht, bestehend aus einem Ethylen-Vinylacetat-Copolymer oder einer mikroporösen Folie auf Basis von Polyethylen oder Polypropylen, gegebenenfalls eine sich anschließende Kleberschicht, und abschließend eine vor Gebrauch zu entfernende Schutzschicht. Die wirkstoffhaltige Schicht ist aus einem Polymer aufgebaut, in das eine Vielzahl von flüssigen Mikroreservoiren eingearbeitet ist. Diese Mikroreservoire enthalten den Wirkstoff.

Wie sich gezeigt hat, ist die wirkstoffhaltige Schicht - obwohl hierin der Wirkstoff in einer flüssigen Zubereitung enthalten ist - selbst bei mechanischer Beschädigung (Schnitte, Risse, Abrieb etc.) absolut auslaufsicher. Der Benutzer ist somit hinsichtlich einer unkontrollierten Freisetzung bzw. einer versehentlichen Überdosierung als Folge einer unbeabsichtigten oder vorsätzlichen Beschädigung der wirkstoffhaltigen Schicht keiner Gefährdung ausgesetzt.

Rein äußerlich unterscheidet sich ein solches Transdermales Therapeutisches System nicht von dem zweiten Haupttyp eines Transdermalen Therapeutischen Systems, einem Matrixsystem.

Bei dem erfindungsgemäßen Transdermalen Therapeutischen System kann erst unter dem Mikroskop die innere Struktur der wirkstoffhaltigen Schicht erkannt werden. Die flüssigen Mikroreservoire sind in die (bevorzugt selbstklebend ausgerüstete) wirkstoffhaltige Schicht in Form kleiner Tröpfchen eingebettet. (Die Tröpfchen nehmen dabei eine angenähert kugelförmige Gestalt an.) Ein Transdermales Therapeutisches System mit einer derart konstruierten wirkstoffhaltigen Schicht soll im weiteren "Mikroreservoirsystem" genannt werden.

Diese flüssigen Mikroreservoire haben einen mittleren Durchmesser von ca. 5 - 50 µm. Sie müssen aber auf jeden Fall kleiner sein als die Dicke der wirkstoffhaltigen Schicht, da ansonsten die wirkstoffhaltige Flüssigkeit austreten könnte. Die Größe der Mikroreservoire kann durch die Wahl geeigneter Flüssigkeiten und die Steuerung bestimmter Parameter bei der Herstellung beeinflußt werden.

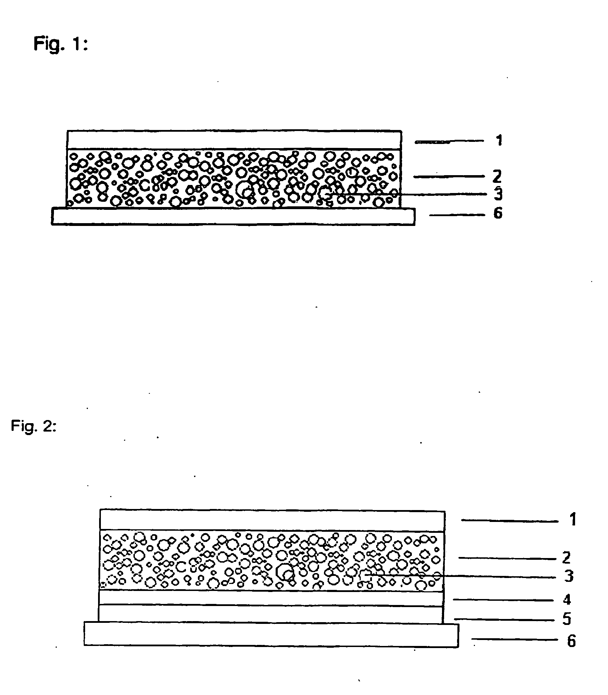

Wie ein Matrixsystem besteht ein Mikroreservoirsystem im einfachsten Fall also aus drei Schichten: einer für den Wirkstoff undurchlässigen Rückschicht, der wirkstoffhaltigen, selbstklebenden Schicht mit den Mikroreservoiren und einer vor Gebrauch zu entfernenden Schutzschicht. Ein solches System gemäß dem Stand der Technik ist in Fig. 1 dargestellt.

Es kann aber unter Umständen nötig sein, auch bei einem Mikroreservoirsystem die von dem Transdermalen Therapeutischen System über einen bestimmten Zeitraum abzugebende Menge des Wirkstoffs zu begrenzen. Dies wird durch eine sich an die wirkstoffhaltige Schicht hautseitig anschließende Membran errreicht, die zur Befestigung auf der Haut zusätzlich mit einer Kleberschicht versehen sein kann. Diese hautseitige Kleberschicht kann bei der Herstellung mit einer begrenzten Menge Wirkstoff versehen werden, die nach Applikation eines solchen Mikroreservoirsystems in einer von der Membran nicht kontrollierbaren Weise an die Haut und damit in den Organismus abgegeben wird. Sinn dieser Maßnahme ist es, die Zeit bis zum Erreichen eines therapeutischen Plasmaspiegels (sog. "lag-time") zu verkürzen. Ein Mikroreservoirsystem mit Membran ist in Fig. 2 abgebildet.

Als Wirkstoff kommen Fentanyl und die fentanylanalogen Derivate Sufentanil, Carfentanyl, Lofentanyl und Alfentanil in Frage. Der Wirkstoff liegt vorzugsweise als freie Base vor; er kann aber auch in Form eines pharmazeutisch annehmbaren Salzes oder als Gemisch der freien Base mit einem pharmazeutisch annehmbaren Salz dieser Base eingesetzt werden. Als Salze kommen z. B. die Hydrochloride, Hydrobromide, Sulfate, Hydrogensulfate, Citrate, Tartrate in Frage.

Wie schon erwähnt besitzen Fentanyl und die fentanylanalogen Derivate einen engen therapeutischen Index. Das heißt, daß die Wirkstoffabgaberate eines Transdermalen Therapeutischen Systems, welches Fentanyl oder ein fentanylanalogen Wirkstoff enthält, sehr exakt kontrolliert werden muß.

Es wurde gefunden, daß das Polymer bzw. die Polymermischung, welches der wirkstoffhaltigen Schicht ihren innerem Zusammenhalt verleiht und in die die Mikroreservoire eingebettet sind, bestimmte Anforderungen bezüglich der Lösekapazität für den Wirkstoff und der Mischbarkeit mit den die Mikroreservoire bildenden Flüssigkeiten erfüllen muß. So sollte die Lösekapazität für den Wirkstoff klein sein damit sich Hauptmenge des Wirkstoffs in den Mikroreservoiren und nicht im Polymer selbst befindet. Zusätzlich sollte das Polymer mit den die Mikroreservoire ausbildenden Flüssigkeiten weitgehend unmischbar sein. Durch diese Maßnahmen wird gewährleistet, daß einerseits die Ausbildung von Mikroreservoiren überhaupt erst möglich ist, und daß andererseits die Lösekapazität der Polymerphase für den Wirkstoff nicht zu hoch ist.

Als geeignete Polymere haben sich hydrophobe Polymere herausgestellt, die vorzugsweise haftklebend sind. Hierzu zählen Polyisobutylene und Silikone (Polysiloxane). Als besonders geeignet haben sich aminresistente Polysiloxane erwiesen. In Löslichkeitsstudien wurde gefunden, daß in solchen Polymeren der Wirkstoff nur eine geringe Löslichkeit besitzt. So besitzt Fentanyl in Form der Base beispielsweise in solchen Polymeren eine Löslichkeit von unter 0,5 Gew.-%.

Solche aminresistenten Polymere werden z. B. von der Fa. Dow Coming hergestellt und unter dem Handelsnamen BIO-PSA vertrieben. Die Klebrigkeit dieser Polymere reicht dabei von nicht klebend, mittelstark bis stark klebend, wobei die geeignete Klebrigkeit auch durch Abmischen der einzelnen Typen bzw. durch Zusatz von niedermolekularen Substanzen wie z. B. Silikonöl eingestellt werden kann.

Der Vorteil der aminresistenten Polysiloxanen ist, daß sie über keine freien Siloxanolgruppen verfügen und deshalb in Gegenwart von basischen Wirkstoffen bzw. Salzen von basischen Wirkstoffen nicht zu Kondensationsreaktionen mit negativen Folgen für die Klebkraft neigen. Zudem ist die Wechselwirkung mit den polaren Gruppen der Wirkstoffmoleküle vermindert.

Als Lösemittel für das Polymer können wenig polare und / oder hydrophobe Lösemittel verwendet werden. Aminresistente Polysiloxane werden in verschiedenen Lösemittelsystemen angeboten. Für die Herstellung von Transdermalen Therapeutischen Systemen im Sinne dieser Erfindung eignet sich als Lösemittel am besten n-Heptan bzw. vergleichbare Kohlenwasserstoffe, da die Flüssigkeiten, die für die Mikroreservoire vorgesehen sind, mit diesen Lösemittel nur schlecht mischbar sind.

Dadurch kann während der Herstellung die Lösung des Wirkstoffs in der Mikroreservoirflüssigkeit in der Lösung des Polysiloxans dispergiert werden und damit schon in der zu beschichtenden Masse die Größe der Mikroreservoire durch die Rührbedingungen eingestellt werden. Im Sinne der vorliegenden Beschreibung ist unter einer Dispersion ein System zu verstehen, welches eine zusammenhängende Phase (die aus dem Polymer aufgebaut wird) und aus den nicht miteinander zusammenhängenden Mikroreservoiren (die aus den Flüssigkeitströpfchen aufgebaut wird) besteht.

Die Flüssigkeit, die einen wichtigen Bestandteil der Mikroreservoire darstellt, sollte sowohl mit Wasser als auch mit organischen Lösemitteln zumindest teilweise mischbar sein. Sie kann deshalb auch als ambiphil bezeichnet werden.

Außerdem sollte die Flüssigkeit über ein gutes Lösevermögen für den Wirkstoff verfügen, um in für Transdermale Therapeutische Systeme üblichen Dicken der wirkstoffhaltigen Schichten von ca. 30 bis 300 µm, entsprechend einem Beschichtungsgewicht von 30 - 300 g/m², die nötige Menge Wirkstoff unterzubringen.

Als besonders geeignet erweisen sich Dipropylenglykol, Diethylenglykolmonoethylether, Diethylenglykoldiethylether, Diethylenglykolmonomethylether, Diethylenglykoldimethylether, 1,3-Butandiol, 2,2-Dimethyl-4-hydroxymethyl-1,3-dioxolan, 2-Pyrrolidon und N-Methylpyrrolidon. Anstelle der Reinsubstanzen können natürlich auch deren Abmischungen genommen werden.

Die gemessenen Sättigungslöslichkeiten von Fentanyl in verschiedenen Flüssigkeiten, die für die Mikroreservoire in Frage kommen, sind in Tabelle 1 dargestellt.

**Tabelle 1: Sättigungslöslichkeit von Fentanyl in verschiedenen Flüssigkeiten**

| **Mikroreservoirflüssigkeit** | **Löslichkeit [ % g/g ]** |
|---|---|
| 1,3-Butandiol | 10 |
| Dipropylenglykol | 18 |
| Transcutol * | 25 |
| Diethylenglykoldiethylether | 26 |
| N-Methylpyrrolidon | 26 |

| | |
|---|---|
| * Diethylenglykolmonoethylether | |

Damit ist in den für die Mikroreservoire vorgesehen Flüssigkeiten die Löslichkeit von Fentanyl in Form der Base um etwa den Faktor 20 - 50 höher als in dem Polysiloxanpolymer. Dies ist mehr als ausreichend um die notwendige Menge Wirkstoff in einer 200 µm Dicke nicht überschreitenden Mikroreservoirmatrix und einem System mit akzeptablen Flächengröße unterzubringen.

Die hohe Löslichkeit von Fentanyl in den für die Mikroreservoire vorgesehenen Flüssigkeiten und die zugleich geringe Löslichkeit in dem Silikonpolymer bewirkt zudem, daß sich der weit überwiegende Teil des Fentanyls tatsächlich in den Mikroreservoiren und nicht in der Polymerphase gelöst befindet.

Vorzugsweise befindet sich vor der Anwendung eines erfindungsgemäßen TTS mehr als 50 % des gesamten im TTS vorhandenen Wirkstoffs in den Mikroreservoiren, da sich ansonsten aufgrund der schlechten Löslichkeit im Polymer zu dicke wirkstoffhaltige Schichten und schlechte Gebrauchseigenschaften für die fertigen Systeme ergeben.

Die Gesamtkonzentration des Wirkstoffs in der wirkstoffhaltigen Schicht liegt lediglich zwischen 2 und 5 Gew.-%, wobei dies dann auch in etwa der Sättigungskonzentration des Wirkstoffs im Polymer entspricht. Dies bedeutet, daß trotz der geringen Konzentration die thermodynamische Aktivität des Wirkstoff maximal, d. h. bei oder knapp unterhalb von 1 ist.

Der Anteil der Mikroreservoire an der wirkstoffhaltigen Schicht kann bis zu 40 Gew.-% betragen, wobei es allerdings vorteilhaft ist, 30 Gew.-% nicht zu überschreiten.

Es hat sich als vorteilhaft erwiesen diesen Flüssigkeiten eine die Viskosität erhöhende Substanz zuzusetzen. Hierbei kann es es sich um Polymere handeln, die befähigt sind, mit der Flüssigkeit ein Gel zu bilden. Beispielhaft genannt und auch in den Beispielen verwendet seien Ethylcellulose und Hydroxypropylcellulose. Durch diese Maßnahme erreicht man eine leichtere Dispergierbarkeit der Flüssigkeit in der Lösung des Polymers und kann auch kleinere Durchmesser der Mikroreservoire erzielen.

Für erfindungsgemäße Mikroreservoirsysteme, die mit einer Membran versehen sind, können entweder mikroporöse Membranen oder sogenannte Verteilungsmembranen eingesetzt werden. Mikroporöse Membranen sind Folien, die mit mikroskopisch kleinen Poren bzw. Kanälen versehen sind. Der Wirkstofftransport erfolgt hier im wesentlichen durch diese Poren bzw. Kanäle, die deshalb mit einem für den Wirkstoff diffusiblen Medium (z. B. einer Flüssigkeit, einem Gas, einem Gel oder einer oder sonstigen Masse) gefüllt sein müssen. Die Anzahl, die innere Oberfläche, die Größe der Poren sowie die physikalischchemischen Eigenschaften der Poren- bzw. Kanalfüllung bestimmt dabei die im wesentlichen die Wirkstoffabgabe (Permeationsrate).

Verteilungsmembranen besitzen keine Poren, d. h. der Wirkstoff muß durch das Membranmaterial selbst hindurchdiffundieren. Die Dicke der Membran, die Löslichkeit und der Diffusionskoeffizient des Wirkstoffs im Membranmaterial bestimmen bei Verwendung solcher Membranen die Wirkstoffabgabe. Als besonders gut geeignete Verteilungsmembranen haben sich Membranen auf Basis von Copolymeren aus Ethylen und Vinylacetat (EVA) erwiesen. Solche Membranen sind in verschiedenen Dicken und unterschiedlicher Zusammensetzung erhältlich. Übliche Dicken bewegen sich zwischen 20 und 150 µm und der Vinylacetatgehalt (VA-Gehalt) zwischen 2 und 25 Gew.-%.

Da der VA-Gehalt einen Einfluß auf die Löslichkeit und den Diffusionskoeffizienten der Wirkstoffe in den EVA-Polymeren hat, ist er bei Verwendung von Membranen aus diesem Material eine weitere wichtige Kenngröße zur Membrancharakterisierung. In den Beispielen wurde eine 50 µm dicke Membran mit einem VA-Gehalt von 9 Gew.-% verwendet. Die unter Verwendung dieser Membran erreichten Permeationsraten können durch die Verwendung von dünneren Membranen bzw. Membranen mit einem höheren VA-Gehalt gesteigert werden. Naturgemäß hat die Verwendung von dickeren Membranen und die Verminderung des VA-Gehalts eine gegenteilige Wirkung.

Die Wirkstoffaufnahme, das heißt die über die Haut tatsächlich in den Blutkreislauf aufgenommene Menge des von dem TTS abgegebenen Wirkstoffs hängt auch von der Permeabilität der Haut ab. Speziell die äußere Hautschicht, das Stratum Comeum, bildet dabei die Hauptbarriere gegen eindringende Wirkstoffe. Diese Barrierefunktion kann durch die Verwendung von sogenannten Enhancem erniedrigt und dadurch die Wirkstoffaufnahme gesteigert werden.

Enhancer sind dem Fachmann bekannt, z. B. aus der Veröffentlichung "Skin penetration enhancers cited in the technical literature" von David W. Osbome und Jill J. Henke, ViroTex Corporation, die im Internet unter http://www.pharmtech.com/technical/osbome/osbome.htm abgerufen werden kann und die zur Vermeidung von Wiederholungen vollinhaltlich Teil der Offenbarung dieser Anmeldung sein soll.

Besonders vorteilhaft können für die Transdermalen Therapeutischen Systeme der vorliegenden Erfindung Fettsäuren, Fettsäureester, Fettalkohole oder Glycerinester als Enhancer verwendet werden, insbesondere, wenn als Wirkstoff Fentanyl eingesetzt wird.

Für die Herstellung der wirkstoffhaltigen Schicht wird der Wirkstoff in der die Mikroreservoire bildenden Flüssigkeit gelöst, und diese Lösung in der Lösung des Polymers dispergiert. Mit dieser Dispersion wird nun ein geeignetes Substrat - üblicherweise eine abhäsiv beschichtete Polyesterfolie - beschichtet und mittels Trocknung das Lösemittel des Polymers entfernt. Die Trocknungsbedingungen sind dabei so zu wählen, daß das Lösemittel der Mikroreservoire nicht oder nur zu einem geringen Teil entfernt wird. Es hat sich gezeigt, daß die Flüssigkeit und das Lösemittel vorzugsweise so ausgewählt werden, daß das Lösemittel eine Siedetemperatur besitzt, die mindestens etwa 30°, besonders bevorzugt mindestens 50° unterhalb der Siedetemperatur der Flüssigkeit liegt.

Der getrocknete Matrixfilm wird nun mit der Rückschicht des Systems - üblicherweise einer ca. 15 - 30 µm dicken wirkstoffundurchlässigen Folie - kaschiert und aus dem so erhaltenen Gesamtlaminat dann die einzelnen Transdermalen Therapeutischen Systeme der Vergleichsbeispiele ausgestanzt.

Die Herstellung entsprechender Mikroreservoirsysteme mit Membranen ist etwas komplizierter, unterscheidet sich aber bezüglich der Beschichtungs-, Laminier- und Kaschierprozesse ebenfalls nicht von der Herstellung bekannter Systeme mit gleicher Schichtenabfolge. In den Beispielen ist die Herstellung von Mikroreservoirsystemen mit und ohne Membran (für Vergleichszwecke) detailliert beschrieben.

Mit drei Transdermalen Therapeutischen Systemen, d. h. Mikroreservoirsystemeh mit und ohne Membran wurden Permeationsstudien unter Verwendung von menschlicher Epidermis und den dem Fachmann bekannten Franz-Diffusionszellen durchgeführt. Die Zusammensetzung und die Ergebnisse sind in den Tabellen 2 bis 5 zusammengefaßt, die Herstellung ist detailliert in den Ausführungsbeispielen beschrieben.

**Tabelle 2: Zusammensetzung der Mikroreservoirsysteme ohne Membran**

| | Zusammensetzung [ % g/ g ] | | |
|---|---|---|---|
| Formulierung | A | B | C |
| Inhaltsstoffe | | | |
| Fentanyl Base | 2,0 | 3,6 | 5,0 |
| BIO-PSA 4301* | 80,0 | 80,0 | 80 |
| 1,3-Butandiol | 17,46 | | |
| Dipropylenglykol | | 16,07 | |
| Transcutol ** | | | 14,4 |
| Hydroxypropylcellulose | 0,54 | 0,33 | |
| Ethylcellulose 100NF | | | 0,6 |
| Beschichtungsgewicht [g/m²] | 130 | 85 | 65 |
| Matrixdicke ca. [ µm ] | 140 | 95 | 75 |

| | | | |
|---|---|---|---|
| * aminresistenter Silikonkleber mit hoher Klebkraft ** Diethylenglykolmonoethylether | | | |

**Tabelle 3: Ergebnisse der Permeationsstudie mit Formulierung A, B und C**

| Formulierung | Kumulierte permeierte Menge Fentanylbase [µg/cm²]* | | | | | mittlere Abgaberate |
|---|---|---|---|---|---|---|
| | 4 h | 8 h | 24 h | 48 h | 72 h | [µg/cm²*h ] |
| A | 1,2 | 9,5 | 84,4 | 194,0 | 275,0 | 3,82 |
| B | 1,12 | 8,87 | 75,80 | 191,00 | 283.00 | 3,93 |
| C | 16,20 | 45,20 | 131,00 | 212,00 | 262,00 | 3,64 |

| | | | | | | |
|---|---|---|---|---|---|---|
| * Mittelwerte aus n = 3 | | | | | | |

**Tabelle 4: Zusammensetzung der Systeme mit Steuermembran**

| | Zusammensetzung [ % g/ g ] | | |
|---|---|---|---|
| Formulierung | D | E | F |
| Inhaltsstoffe | | | |
| Reservoirschicht | | | |
| Fentanyl Base | 2,0 | 3,6 | 5,0 |
| BIO-PSA 4301 * | 80,0 | 80,0 | 80 |
| 1,3 Butandiol | 17,46 | | |
| Dipropylenglykol | | 16,07 | |
| Transcutol ** | | | 14,4 |
| Hydroxypropylcellulose | 0,54 | 0,33 | |
| Ethylcellulose 100NF | | | 0,6 |
| | | | |
| Beschichtungsgewicht [ g/m² ] | 130 | 85 | 65 |
| | | | |
| Steuermembran | EVA-Membran 50 µm, 9 % VA | | |
| | | | |
| ***Hautkontaktsschicht*** | | | |
| BIO-PSA 4301 * | 100 | 100 | 100 |
| Beschichtungsgewicht [g/ m²] | 35 | 35 | 35 |

| | | | |
|---|---|---|---|
| * aminresistenter Silikonkleber mit hoher Klebkraft ** Diethylenglykolmonoethylether | | | |

**Tabelle 5: Ergebnisse der Permeationsstudie mit Formulierung D, E und F**

| Formulie rung | Kumulierte permeierte Menge Fentanylbase [µg/cm²] * | | | | | mittlere Abgaberate |
|---|---|---|---|---|---|---|
| | 4 h | 8 h | 24 h | 48 h | 72 h | [ µg/cm²*h ] |
| D | 1,4 | 4,9 | 26,2 | 64,0 | 99,8 | 1,39 |
| E | 2,7 | 8,1 | 37,0 | 85,0 | 129,0 | 1,79 |
| F | 0,8 | 4,7 | 45,3 | 114,0 | 166,0 | 2,31 |

| | | | | | | |
|---|---|---|---|---|---|---|
| * Mittelwerte aus n = 3 | | | | | | |

Beim Vergleich der Permeationsergebnisse der Mikroreservoirsysteme mit und ohne Membran ist zu erkennen, daß die nach 72 Stunden permeierte Menge Wirkstoff trotz gleicher Zusammensetzung der wirkstoffhaltigen Schichten bei den Membransystemen deutlich geringer ist. Dies ist auf die steuernde Wirkung der Membran zurückzuführen, die die Wirkstoffabgabe unabhängig von der jeweiligen Beschaffenheit der Haut auf einen Maximalwert begrenzt.

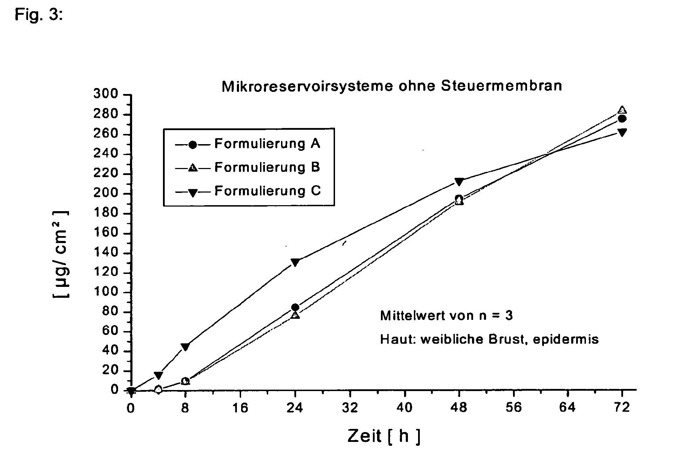

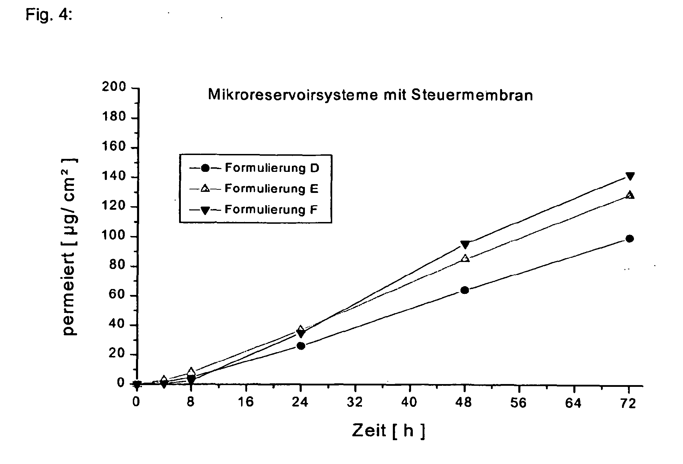

In der graphischen Darstellung (Fig. 3 und Fig. 4) ist auch zu erkennen, daß durch die Verwendung einer Membran das Permeationsprofil linearer verläuft und damit auch die Wirkstoffaufnahme *in vivo* über den Anwendungszeitraum gleichmäßiger erfolgt. Besonders gut ist dies zu erkennen bei Formulierung C, die die höchste Permeationsrate zeigt.

Das auf dem Markt befindliche TTS (Durogesic^{®}) ist in 4 Stärken erhältlich mit mittleren Abgaberaten von 25, 50, 75 und 100 µm/Stunde. Mit diesen Angaben und den Ergebnissen der Permeationsstudien können die Systemflächen einfach errechnet werden. Die Ergebnisse sind in Tabelle 6 zusammengefaßt.

**Tabelle 6: Errechnete Systemflächen der Formulierungen A-F**

| Abgaberate | berechnete Flächengrößen [ cm² ] | | | | | |
|---|---|---|---|---|---|---|
| | A | B | C | D | E | F |
| 25 µm/ h | 6,5 | 6,4 | 6,9 | 18,0 | 14,0 | 11,8 |
| 50 µm/ h | 13,0 | 12,8 | 13,8 | 36,0 | 28,0 | 23,6 |
| 75 µm/ h | 19,5 | 19,2 | 20,7 | 54,0 | 42,0 | 35,4 |
| 100 µm/ h | 26,0 | 25,6 | 27,6 | 72,0 | 56,0 | 47,2 |

Die errechneten Flächen liegen alle in einem akzeptablen Bereich. Die Größe der Mikroreservoirsysteme mit Membran kann durch die Verwendung von dünneren Membranen bzw. Membranen mit einem höheren VA-Gehalt noch verkleinert werden, wobei allerdings dann auch die Kontrolle der Wirkstoffabgabe durch die Membran weniger wirksam wird.

Mikroreservoirsysteme zeigen also sehr gute Permeationsraten, die zu Vergleichszwecken ohne Steuermembran zu flächenmäßig kleinen und angenehm zu tragenden TTS führen. Gleichzeitig ist eine Gefährdung des Patienten durch eine zu hohe Wirkstoffaufnahme infolge einer Leckage absolut unmöglich.

Bei Mikroreservoirsystemen mit Membranen ergeben sich durch die die Wirkstoffabgabe begrenzende Membransteuerung größere aber immer noch akzeptable Pflastergrößen.

Insgesamt stellen damit Mikroreservoirsysteme für Fentanyl und die fentanylanalogen Derivate damit bezüglich des Tragekomforts und der Patientensicherheit einen bedeutenden Fortschritt gegenüber dem bekannten Stand der Technik dar.

Die folgenden Herstellungsbeispiele beschreiben die Herstellung von Mikroreservoirsysteme mit und ohne Membranen.

### Vergleichsbeispiel 1: Mikroreservoirsystem mit 1,3-Butandiol als Flüssigkeit, Formulierung A

1 g Fentanylbase wird in 9 g mit 3 % Hydroxypropylcellulose angedicktem 1,3-Butandiol gelöst. Zu dieser Lösung werden 54,8 g einer 73 %-igen Lösung eines aminresistenten Silikonklebers in n-Heptan (BIO-PSA 4301, Dow Corning) gegeben und die Wirkstofflösung durch schnelles Rühren in der Lösung des Silikonklebers dispergiert. Die Dispersion wird nun mit einem Rakel auf eine abhäsiv beschichtete Folie, die spätere vor Gebrauch zuentfemende Schutzschicht (Scotchpak 1022, 3M) in einer Dicke beschichtet, die nach der Entfernung des n-Heptans durch 15-minütiges Trocknen bei 30°C ein Behsichtungsgewicht von 135 g/m² ergibt. Der getrocknete Film wird nun mit der wirkstoffundurchlässigen Rückschicht (Scotchpak 1220, 3M) kaschiert und aus dem resultierenden Gesamtlaminat die fertigen Transdermalen Therapeutischen Systeme ausgestanzt.

### Vergleichsbeispiel 2: Mikroreservoirsystem mit Dipropylenglykol als Flüssigkeit, Formulierung B

1 g Fentanylbase wird in 4,6 g mit 2 % Hydroxypropylcellulose angedicktem Dipropylenglykol gelöst. Zu dieser Lösung werden 30,5 g einer 73 %-igen Lösung eines aminresistenten Silikonklebers in n-Heptan (BIO-PSA 4301, Dow Coming) gegeben und die Wirkstofflösung durch schnelles Rühren in der Lösung des Silikonklebers-dispergiert. Die Dispersion wird nun mit einem Rakel auf eine abhäsiv beschichtete Folie, die spätere vor Gebrauch zu entfernende Schutzschicht (Scotchpak 1022, 3M) in einer Dicke beschichtet, die nach der Entfernung des n-Heptans durch 15-minütiges Trocknen bei 30 °C ein Beschichtungsgewicht von 85 g/m² ergibt. Der getrocknete Film wird nun mit der wirkstoffundurchlässigen Rückschicht (Scotchpak 1220, 3M) kaschiert und aus dem resultierenden Gesamtlaminat die fertigen Transdermalen Therapeutischen Systeme ausgestanzt.

### Vergleichsbeispiel 3: Mikroreservoirsystem mit Transcutol als Flüssigkeit, Formulierung C

1 g Fentanylbase wird in 3 g mit 4 % Ethylcellulose angedicktem Transcutol gelöst. Zu dieser Lösung werden 22 g einer 73 %-igen Lösung eines aminresistenten Silikonklebers in n-Heptan (BIO-PSA 4301, Dow Coming) gegeben und die Wirkstofflösung durch schnelles Rühren in der Lösung des Silikonklebers dispergiert. Die Dispersion wird nun mit einem Rakel auf eine abhäsiv beschichtete Folie, die spätere vor Gebrauch zu entfernende Schutzschicht (Scotchpak 1022, 3M) in einer Dicke beschichtet, die nach der Entfernung des n-Heptans durch 15-minütiges Trocknen bei 30 °C ein Beschichtungsgewicht von 65 g/m² ergibt. Der getrocknete Film wird nun mit der wirkstoffundurchlässigen Rückschicht (Scotchpak 1220, 3M) kaschiert und aus dem resultierenden Gesamtlaminat die fertigen Transdermalen Therapeutischen Systeme ausgestanzt.

### Beispiele 4 - 6: Mikroreservoirsysteme mit Membranen, Formulierungen D, E und F

Die Lösung eines aminresistenten Silikonklebers (BIO-PSA 4301, Dow Coming) wird mit einem Rakel auf eine abhäsiv beschichtete Folie (Scotchpak 1022, 3M) in einer Dicke beschichtet, die nach dem Entfernen des n-Heptans durch 15 minütiges Trocknen bei 30 °C ein Beschichtungsgewicht von 20 g/m² ergibt. Der getrocknete Film wird mit der Membran (EVA, 50 µm, 9 %VA, 3M) kaschiert.

Von den Laminaten gemäß Beispiel 1 - 3 wird die Schutzschicht entfernt und das Laminat, bestehend aus wirkstoffhaltiger Schicht und Rückschicht, auf diese Membran kaschiert. Aus dem resultierenden Gesamtlaminat (Formulierungen D, E und F) werden dann die fertigen Transdermalen Therapeutischen Systeme ausgestanzt.

In den Figuren haben die dargestellen Elemente folgende Bedeutung:
1 = Rückschicht
2 = wirkstoffhaltige Schicht mit Mikroreservoiren
3 = Mikroreservoir
4 = Steuermembran
5 = Hautkontaktschicht
6 = wieder entfernbare Schutzschicht

## Patentansprüche

1. Transdermales Therapeutisches System (TTS) mit erhöhter Sicherheit hinsichtlich einer versehentlichen Verabreichung einer Überdosis des Wirkstoffes umfassend eine wirkstoffundurchlässige Rückschicht, eine wirkstoffhaltige Schicht, wobei der Wirkstoff Fentanyl, Sufentanil, Carfentanyl, Lofentanyl und/oder Alfentanil und/oder ein Salz von Fentanyl, Sufentanil, Carfentanyl, Lofentanyl und/oder Alfentanil ist und wobei die wirkstoffhaltige Schicht ein Polymer oder eine Polymermischung mit darin dispergierten Mikroreservoiren umfasst, eine sich hautseitig anschließende Membranschicht, bestehend aus einem Ethylen-Vinylacetat-Copolymer oder einer mikroporösen Folie auf Basis von Polyethylen oder Polypropylen, gegebenenfalls eine sich anschließende Kleberschicht, und abschließend eine vor Gebrauch zu entfernende Schutzschicht.

2. TTS gemäß Anspruch 1, **dadurch gekennzeichnet, dass** die Membranschicht aus einem Ethylen-Vinylacetat-Copolymer mit einem Vinylacetat-Anteil von 2 bis 25 Gew.-% besteht und eine Dicke zwischen 20 und 150 µm aufweist.

3. TTS gemäß Anspruch 1 oder 2, **dadurch gekennzeichnet, dass** der Anteil der Mikroreservoire an der wirkstoffhaltigen Schicht bis zu 40 Gew.-%, vorzugsweise weniger als 30 Gew.-% beträgt.

4. TTS gemäß einem oder mehreren der vorhergehenden Ansprüche, **dadurch gekennzeichnet, dass** die Kleberschicht mit einer begrenzten Menge Wirkstoff versehen wird, die nach Applikation in einer von der Membran nicht kontrollierbaren Weise an die Haut abgegeben wird.

5. TTS nach einem oder mehreren der vorangehenden Ansprüche, **dadurch gekennzeichnet, dass** das Polymer oder die Polymermischung aus der Gruppe der Polyisobutylene und Silikone ausgewählt ist.

6. TTS nach einem oder mehreren der vorangehenden Ansprüche **dadurch gekennzeichnet, dass** das Polymer ein aminresistentes Polysiloxan ist.

7. TTS nach einem oder mehreren der vorangehenden Ansprüche, **dadurch gekennzeichnet, dass** die Mikroreservoire eine Flüssigkeit enthalten.

8. TTS nach einem der vorangehenden Ansprüche, **dadurch gekennzeichnet, dass** der Wirkstoff in den Mikroreservoiren vollständig gelöst vorliegt.

9. TTS nach einem der vorangehenden Ansprüche, **dadurch gekennzeichnet, dass** die Mikroreservoire einen mittleren Durchmesser von 5 bis 50 µm besitzen.

10. TTS nach einem oder mehreren der vorangehenden Ansprüche, **dadurch gekennzeichnet, dass** mindestens 50 % des im TTS enthaltenen Wirkstoffs in den Mikroreservoiren enthalten ist.

11. TTS nach einem oder mehreren der vorangehenden Ansprüche, **dadurch gekennzeichnet, dass** die Flüssigkeit Dipropylenglykol, Diethylenglykolmonoethylether, Diethylenglykoldiethylether, Diethylenglykolmonomethylether, Diethylenglykoldimethylether, 1,3-Butandiol, 2,2-Dimethyl-4-hydroxymethyl-1,3-dioxolan, 2-Pyrrolidon oder N-Methylpyrrolidon oder eine Kombination davon enthält.

12. TTS nach einem oder mehreren der vorangehenden Ansprüche, **dadurch gekennzeichnet, dass** die Flüssigkeit einen die Viskosität erhöhenden Zusatzstoff enthält, vorzugsweise Ethylcellulose oder Hydroxypropylcellulose.

13. TTS nach einem oder mehreren der vorangehenden Ansprüche, **dadurch gekennzeichnet, dass** die Konzentration des Wirkstoffs in der wirkstoffhaltigen Schicht unterhalb von 5 Gew.-%, bevorzugt unterhalb von 4 Gew.-% ist.

14. TTS nach einem oder mehreren der vorangehenden Ansprüche, **dadurch gekennzeichnet, dass** das Flächengewicht der wirkstoffhaltigen Schicht zwischen 30 und 300 g/m² liegt.

15. TTS gemäß einem oder mehreren der vorangehenden Ansprüche, **dadurch gekennzeichnet, dass** die wirkstoffhaltige Schicht zusätzlich eine die Permeationsrate durch menschliche Haut verbessemde Substanz (Enhancer) enthält.

16. TTS gemäß Anspruch 15, **dadurch gekennzeichnet, dass** die die Permeationsrate durch menschliche Haut verbessemde Substanz zur Gruppe der Fettsäuren, Fettsäureester, Fettalkohole oder Glycerinester gehört.

17. TTS gemäß einem oder mehreren der vorangehenden Ansprüche, **dadurch gekennzeichnet, dass** es eine 50 µm dicke Membran aus einem Ethylen-Vinylacetat-Copolymer mit einem Vinylacetat-Anteil von 9 Gew.-% besitzt.

18. Verfahren zur Herstellung eines Transdermalen Therapeutischen Systems (TTS) umfassend eine wirkstoffundurchlässige Rückschicht, eine wirkstoffhaltige Schicht, und eine vor Gebrauch zu entfernende Schutzschicht, wobei die wirkstoffhaltige Schicht ein Polymer oder eine Polymermischung mit darin dispergierten flüssigen Mikroreservoiren umfasst, **dadurch gekennzeichnet, dass**
a) der Wirkstoff Fentanyl, Sufentanil, Carfentanyl, Lofentanyl und/oder Alfentanil und/oder ein Salz von Fentanyl, Sufentanil, Carfentanyl, Lofentanyl und/oder Alfentanil in einer die Mikroreservoire bildenden Flüssigkeit gelöst wird,
b) die Lösung von Schritt a) in der Lösung eines Polymers oder einer Polymermischung dispergiert wird,
c) ein geeignetes Substrat mit der in Schritt b) erhaltenen Dispersion beschichtet wird,
d) mittels Trocknung das Lösemittels des Polymers oder der Polymermischung entfernt wird, so dass die die Mikroreservoire bildende Flüssigkeit nicht oder nur zu einem geringen-Teil entfernt wird, und
e) die in Schritt d) hergestellte wirkstoffhaltige Schicht mit einer Membran kaschiert wird.

19. Verfahren gemäß Anspruch 18, **dadurch gekennzeichnet, dass** das Lösemittel des Polymers oder der Polymermischung eine Siedetemperatur besitzt, die mindestens 30°, bevorzugt mindestens 50° unterhalb der Siedetemperatur der die Mikroreservoire bildenden Flüssigkeit liegt.

## Claims

1. Transdermal therapeutic system (TTS) with increased security in respect of inadvertent administration of an overdose of the active substance, comprising an active substance impermeable backing layer, an active substance layer, the active substance being fentanyl, sulfentanyl, carfentanyl, lofentanyl and/or alfentanyl and/or a salt of fentanyl, sulfentanyl, carfentanyl, lofentanyl and/or alfentanyl, the active substance layer comprising a polymer or a polymer mixture having microreservoirs dispersed therein, a membrane layer which follows on the skin side, composed of an ethylene-vinyl acetate polymer or of a microporous film based on polyethylene or polypropylene, optionally a subsequent adhesive layer, and, finally, a protective layer which is to be removed before use.

2. TTS according to Claim 1, **characterized in that** the membrane layer is composed of an ethylene-vinyl acetate copolymer having a vinyl acetate fraction of 2% to 25% by weight and has a thickness of between 20 and 150 µm.

3. TTS according to Claim 1 or 2, **characterized in that** the fraction of the microreservoirs as a proportion of the active substance layer is up to 40% by weight, preferably less than 30% by weight.

4. TTS according to one or more of the preceding claims, **characterized in that** the adhesive layer is provided with a limited amount of active substance, which following application is delivered to the skin in a way which is not controllable by the membrane.

5. TTS according to one or more of the preceding claims, **characterized in that** the polymer or the polymer mixture is selected from the group consisting of polyisobutylenes and silicones.

6. TTS according to one of more of the preceding claims, **characterized in that** the polymer is an amine-resistant polysiloxane.

7. TTS according to one or more of the preceding claims, **characterized in that** the microreservoirs contain a liquid.

8. TTS according to any of the preceding claims, **characterized in that** the active substance is present fully dissolved in the microreservoirs.

9. TTS according to any of the preceding claims, **characterized in that** the microreservoirs possess an average diameter of 5 to 50 µm.

10. TTS according to one or more of the preceding claims, **characterized in that** at least 50% of the active substance present in the TTS is contained in the microreservoirs.

11. TTS according to one or more of the preceding claims, **characterized in that** the liquid comprises dipropylene glycol, diethylene glycol monoethyl ether, diethylene glycol diethyl ether, diethylene glycol monomethyl ether, diethylene glycol dimethyl ether, 1,3-butanediol, 2,2-dimethyl-4-hydroxymethyl-1,3-dioxolane, 2-pyrrolidone or N-methylpyrrolidone or a combination thereof.

12. TTS according to one or more of the preceding claims, **characterized in that** the liquid comprises a viscosity-increasing additive, preferably ethylcellulose or hydroxypropylcellulose.

13. TTS according to one or more of the preceding claims, **characterized in that** the concentration of the active substance in the active substance layer is below 5% by weight, preferably below 4% by weight.

14. TTS according to one or more of the preceding claims, **characterized in that** the weight of the active substance layer per unit area is between 30 and 300 g/m²_{.}

15. TTS according to one or more of the preceding claims, **characterized in that** the active substance layer further comprises a substance which enhances the rate of permeation through human skin (permeation enhancer).

16. TTS according to Claim 15, **characterized in that** the substance which enhances the rate of permeation through human skin belongs to the group of fatty acids, fatty acid esters, fatty alcohols or glycerol esters.

17. TTS according to one or more of the preceding claims, **characterized in that** it possesses a membrane 50 µm thick, comprising an ethylene-vinyl acetate copolymer having a vinyl acetate fraction of 9% by weight.

18. Method of producing a transdermal therapeutic system (TTS) comprising an active substance impermeable backing layer, an active substance layer, and a protective layer, which is to be removed before use, the active substance layer comprising a polymer or a polymer mixture having liquid microreservoirs dispersed therein, **characterized in that**
a) the active substance fentanyl, sulfentanyl, carfentanyl, lofentanyl and/or alfentanyl and/or a salt of fentanyl, sulfentanyl, carfentanyl, lofentanyl and/or alfentanyl is dissolved in a liquid which forms the microreservoirs,
b) the solution from step a) is dispersed in the solution of a polymer or of a polymer mixture,
c) a suitable substrate is coated with the dispersion obtained in step b),
d) the solvent of the polymer or of the polymer mixture is removed by drying, and so the liquid forming the microreservoirs is not removed or is removed only to a small extent, and
e) the active substance layer produced in step d) is laminated with a membrane.

19. Method according to Claim 18, **characterized in that** the solvent of the polymer or of the polymer mixture possesses a boiling temperature which is at least 30°, preferably at least 50°, below the boiling temperature of the liquid which forms the microreservoirs.

## Revendications

1. Système thérapeutique transdermique (STT) avec une sécurité élevée en ce qui concerne une administration par erreur d'une surdose de la substance active, comprenant une couche dorsale imperméable à la substance active, une couche contenant une substance active, la substance active étant le Fentanyl, le Sulfentanil, le Carfentanil, le Lofentanil et/ou l'Alfentanil et/ou un sel du Fentanyl, du Sulfentanil, du Carfentanil, du Lofentanil et/ou de l'Alfentanil et la couche contenant une substance active comprenant un polymère ou un mélange de polymères avec des microréservoirs qui y sont dispersés, ensuite, côté peau, une couche membranaire, constituée par un copolymère d'éthylène-acétate de vinyle ou une feuille microporeuse à base de polyéthylène ou de polypropylène, ensuite le cas échéant une couche adhésive, et enfin une couche de protection à enlever avant emploi.

2. STT selon la revendication 1, **caractérisé en ce que** la couche membranaire est constituée par un copolymère d'éthylène-acétate de vinyle avec une proportion d' acétate de vinyle de 2 à 25% en poids et présente une épaisseur entre 20 et 150 µm.

3. STT selon la revendication 1 ou 2, **caractérisé en ce que** la proportion de microréservoirs dans la couche contenant la substance active est de jusqu'à 40% en poids, de préférence inférieure à 30% en poids.

4. STT selon l'une ou plusieurs des revendications précédentes, **caractérisé en ce que** la couche adhésive est pourvue d'une quantité limitée de substance active, qui est cédée à la peau d'une manière non contrôlable par la membrane après l'application.

5. STT selon l'une ou plusieurs des revendications précédentes, **caractérisé en ce que** le polymère ou le mélange de polymères est choisi dans le groupe des polyisobutylènes et des silicones.

6. STT selon l'une ou plusieurs des revendications précédentes, **caractérisé en ce que** le polymère est un polysiloxane résistant aux amines.

7. STT selon l'une ou plusieurs des revendications précédentes, **caractérisé en ce que** les microréservoirs contiennent un liquide.

8. STT selon l'une quelconque des revendications précédentes, **caractérisé en ce que** la substance active se trouve sous forme totalement dissoute dans les microréservoirs.

9. STT selon l'une quelconque des revendications précédentes, **caractérisé en ce que** les microréservoirs présentent un diamètre moyen de 5 à 50 µm.

10. STT selon l'une ou plusieurs des revendications précédentes, **caractérisé en ce qu'**au moins 50% de la substance active contenue dans le STT sont contenus dans les microréservoirs.

11. STT selon l'une ou plusieurs des revendications précédentes, **caractérisé en ce que** le liquide contient du dipropylèneglycol, du diéthylèneglycolmonoéthyléther, du diéthylèneglycoldiéthyléther, du diéthylèneglycolmonométhyléther, du diéthylèneglycoldiméthyléther, du 1,3-butanediol, du 2,2-diméthyl-4-hydroxyméthyl-1,3-dioxolane, de la 2-pyrrolidone ou de la N-méthylpyrrolidone ou une combinaison de ceux-ci.

12. STT selon l'une ou plusieurs des revendications précédentes, **caractérisé en ce que** le liquide contient un additif augmentant la viscosité, de préférence de l'éthylcellulose ou de l'hydroxypropylcellulose.

13. STT selon l'une ou plusieurs des revendications précédentes, **caractérisé en ce que** la concentration de la substance active dans la couche contenant la substance active est inférieure à 5% en poids, de préférence inférieure à 4% en poids.

14. STT selon l'une ou plusieurs des revendications précédentes, **caractérisé en ce que** le poids surfacique de la couche contenant la substance active est situé entre 30 et 300 g/m².

15. STT selon l'une ou plusieurs des revendications précédentes, **caractérisé en ce que** la couche contenant la substance active contient en outre une substance améliorant le taux de perméation dans la peau humaine (Enhancer).

16. STT selon la revendication 15, **caractérisé en ce que** la substance améliorant le taux de perméation dans la peau humaine appartient au groupe des acides gras, des esters d'acides gras, des alcools gras ou des esters de glycérol.

17. STT selon l'une ou plusieurs des revendications précédentes, **caractérisé en ce qu'**il présente une membrane d'une épaisseur de 50 µm en un copolymère d'éthylène-acétate de vinyle avec une proportion d'acétate de vinyle de 9% en poids.

18. Procédé pour la confection d'un système thérapeutique transdermique (STT) comprenant une couche dorsale imperméable à la substance active, une couche contenant une substance active et une couche de protection à éliminer avant l'emploi, la couche contenant la substance active comprenant un polymère ou un mélange de polymères avec des microréservoirs liquides qui y sont dispersés, **caractérisé en ce que**
a) la substance active Fentanyl, Sulfentanil, Carfentanil, Lofentanil et/ou Alfentanil et/ou un sel de Fentanyl, de Sulfentanil, de Carfentanil, de Lofentanil et/ou d'Afentanil est dissoute/dissous dans un liquide formant les microréservoirs,
b) la solution de l'étape a) est dispersée dans la solution d'un polymère ou d'un mélange de polymères,
c) un substrat approprié est revêtu avec la dispersion obtenue dans l'étape b),
d) le solvant du polymère ou du mélange de polymères est éliminé par séchage, de manière telle que le liquide formant les microréservoirs n'est pas éliminé ou seulement à raison d'une petite partie, et
e) la couche contenant la substance active préparée dans l'étape d) est contrecollée par une membrane.

19. Procédé selon la revendication 18, **caractérisé en ce que** le solvant du polymère ou du mélange de polymères présente une température d'ébullition, qui est inférieure d'au moins 30°, de préférence d'au moins 50° à la température d'ébullition du liquide formant les microréservoirs.
